(19) Europäisches Patentamt
European Patent Office
Office européen des brevets

(11) Veröffentlichungsnummer: **0 112 485**
**B1**

(12) # EUROPÄISCHE PATENTSCHRIFT

(45) Veröffentlichungstag der Patentschrift: **27.04.88**

(21) Anmeldenummer: **83111479.8**

(22) Anmeldetag: **17.11.83**

(51) Int. Cl.⁴: **A 61 K 9/06,** A 61 K 7/16, A 61 K 31/415, A 61 K 47/00

(54) **Antimykotische Mittel in Gelform zur Behandlung von Pilzinfektionen der Mundhöhle.**

(30) Priorität: **25.11.82 DE 3243546**

(43) Veröffentlichungstag der Anmeldung: **04.07.84 Patentblatt 84/27**

(45) Bekanntmachung des Hinweises auf die Patenterteilung: **27.04.88 Patentblatt 88/17**

(84) Benannte Vertragsstaaten: **AT BE CH DE FR GB IT LI NL SE**

(56) Entgegenhaltungen:
**EP-A-0 048 616**
**EP-A-0 055 396**
**BE-A-626 627**
**FR-A-2 225 167**

(73) Patentinhaber: **BAYER AG, Konzernverwaltung RP Patentabteilung, D-5090 Leverkusen 1 Bayerwerk (DE)**

(72) Erfinder: **von Bittera, Miklos, Max- Scheler-Strasse 7, D-5090 Leverkusen 3 (DE)**
Erfinder: **Büchel, Karl Heinz, Prof. Dr., Dabringhausener Strasse 42, D-5093 Burscheid (DE)**
Erfinder: **Plempel, Manfred, Dr., Zwengenberger Strasse 3c, D-5657 Haan (DE)**
Erfinder: **Regel, Erik, Dipl.- Ing., Untere Bergerheide 26, D-5600 Wuppertal 1 (DE)**

**EP 0 112 485 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neuartige antimykotische Mittel in Gelform zur Behandlung von Pilzinfektionen der Mundhöhle, die eine Depot-Wirkung, gute Hafteigenschaften und eine höhere Bioverfügbarkeit der Wirkstoffe aufweisen und dadurch eine Kurzzeittherapie ermöglichen.

Für die Behandlung von Infektionen, vorwiegend durch Sprosspilze, der Mundhöhle, sind bereits Zubereitungen von antimykotischen Verbindungen bekannt geworden. Mit diesen Zubereitungen wurden für eine vollständige Sanierung 10 bis 21 Tage Therapiezeit benötigt.

Um zu einer Verkürzung der Therapiedauer zu kommen, benötigt man, besonders zur Eliminierung der Keime, bzw. um eine mykologische Sanierung zu erzielen, eine gewisse Depot-Wirkung und eine höhere Bioverfügbarkeit der Wirkstoffe. Dafür sind die bekannten Formulierungen nur begrenzt geeignet. Wenn man nun ohne weitere Erhöhung der Wirkstoffkonzentration eine Verkürzung der Therapiedauer erreichen will, muß man für eine optimale Bioverfügbarkeit des Wirkstoffes Sorge tragen.

Erforderlich waren daher Formulierungen, die einerseits nach Auftragen auf die Mundschleimhaut eine ausreichende Haftfähigkeit aufweisen, andererseits den in der Formulierung enthaltenen Wirkstoff ausreichend, auch in Speichel gelöst, freisetzen können.

Aus der EP-A 55 396 sind bereits antimykotische Mittel bekannt. Die bekannten Mittel werden in Form von Flüssig-Pflastern formuliert, d.h. die in flüssiger Form aufgetragene Formulierung bildet auf der Haut einen elastischen Film. Aus der EP-A 48 616 sind Mischungen von Cellulase-freiem Xanthan Gum mit diversen Cellulosen bekannt. Solche Mischungen werden als Verdicker eingesetzt, z. B. bei Zahnpasten oder in der Getränke-Industrie.

Es wurde nun gefunden, daß solche Formulierungen antimykotischer Wirkstoffe, die als Gelbildner einen Celluloseether, insbesondere Hydroxypropylcellulose, Natriumalginat oder Propylenglykolalginat und außerdem die üblichen Formulierungshilfsstoffe enthalten, optimale Hafteigenschaften und eine optimale Freisetzung des Wirkstoffes und damit eine verkürzte Therapiedauer durch das Erreichen von fungiziden Konzentrationen des Wirkstoffes ermöglichen. Dieser Effekt wird dadurch erreicht, daß die Bioverfügbarkeit der in den Formulierungen enthaltenden Wirkstoffe durch adhärierende Eigenschaften erhöht werden und dadurch die Wirkstoff-Freisetzung in Speichel gesteigert werden kann.

Insbesondere wurde gefunden, daß solche Formulierungen antimykotischer Derivate, die die oben genannten Gelbildner und zusätzlich Xanthan Gum als Stabilisator und/oder Gelbildner enthalten, die Wärmestabilität der Formulierungen und damit die Langzeithaltbarkeit wesentlich verbessern. Formulierungen ohne Zusatz von Xanthan Gum, einem hochmolekularen Polysaccharid, sind nur bis 30°C haltbar, über 30°C tritt nach kurzer Zeit eine Phasentrennung auf, d.h. die Gelstruktur wird irreversibel zerstört. Formulierungen, die neben den Gelbildnern zusätzlich in geringen Mengen Xanthan Gum enthalten, sind bis 500°C stabil und dadurch wird die Haltbarkeit solcher Zubereitungen wesentlich erhöht.

Die so hergestellten Mittel besitzen auch andere außergewöhnliche Eigenschaften:

Die Viskosität wird von Temperaturschwankungen kaum beeinflußt; die adhärierenden Eigenschaften der Formulierungen werden verbessert.

Die Geschmacksentfaltung wird verbessert und ein gutes Mundgefühl erzielt.

Wirkstoffe, die in dieser Weise formuliert werden können, sind alle antimykotisch wirksamen Derivate, insbesondere Imidazol- und Triazolderivate. Sie sind in den erfindungsgemäßen Mitteln in Mengen von 0,05 - 1 %, vorzugsweise 0,1 - 1 %, vorhanden.

Beispielsweise seien die Verbindungen der nachstehenden Formeln genannt:

I 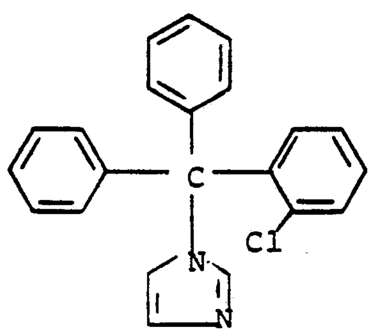 Clotrimazol

II 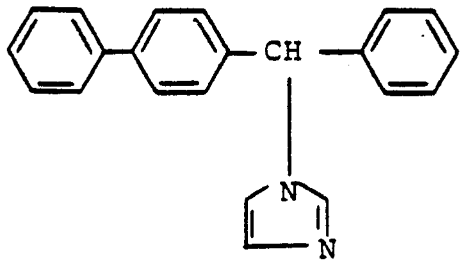 Bifonazol

III 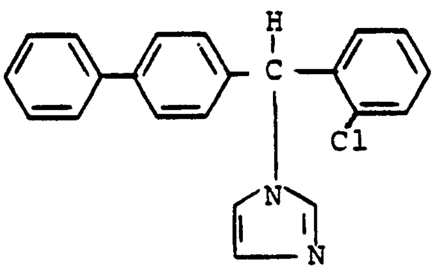 Lombazol

Zahlreiche weitere antimykotisch wirksame Azolderivate sind bekannt aus der DE-OS 2 430 039. Sie können ebenfalls in den erfindungsgemäßen Mitteln als Wirkstoffe dienen.

Als Gelbildner kommen solche makromolekularen Verbindungen in Frage, die sich sowohl in Wasser als auch in organischen Lösungsmitteln lösen bzw. anquellen können. Vor allem seien hier Celluloseether genannt, von denen 2,5 bis 17,5 % benötigt werden. Besonders geeignet ist Hydroxypropylcellulose, insbesondere solche mit Molgewichten von 1.000 000 bis 60.000. Folgt man einer Einteilung der makromolekularen Hilfsstoffe, (Keipert et al., Die Pharmazie 28, 145-183 (1973)), so kommen vor allem ionische Makromoleküle in deren Salzform zur Anwendung. Dies sind unter anderem Natriumcarboxymethylcellulose, Polyacrylsäure, Polymethacrylsäure und deren Salze, Natriumamylopektinsemiglykolat, Alginsäure und Propylenglykol-Alginat als Natriumsalz, arabisches Gummi, Guar-Gummi.

Amphotere Makromoleküle wie Protein-Derivate, z. B. Gelatine sind ebenso geeignet wie nichtionische Polymere, z. B. Methylcellulose, Hydroxypropylcellulose und lösliche Stärken, die obige Anforderungen erfüllen.

Als Gelbildner, die auch stabilisierend wirken kommt Xanthan Gum, ein langkettiges lineares hochmolekulares Polysaccharid mit einem Molekulargewicht von mehr als einer Million, in Betracht. Von solchen Stabilisatoren werden 0,1 - 1,5 % benötigt.

Als Lösungsmittel sind Wasser und auch alle mit Wasser mischbaren Lösungsmittel geeignet. In Betracht kommen z. B. Alkanole wie Ethanol und Isopropylalkohol, Benzylalkohol, Propylenglykol, u.a..

Als besonders stabile Gel-Kombinationsgrundlagen wurden solche makromolekularen Verbindungen gefunden wie z. B. Hydroxypropylcellulose (Molekulargewicht 60 000-1 000 000) mit einem linearen hochmolekularen Polysaccharid (Molekulargewicht wahrscheinlich 2 000 000).

Als Stabilisator und/oder Gelbildner kommt Xanthan Gum (Keltrol®, Kelzon®) der Firma Kelco in Frage. Dabei handelt es sich um das hochmolekulare natürliche Kohlehydrat der Formel

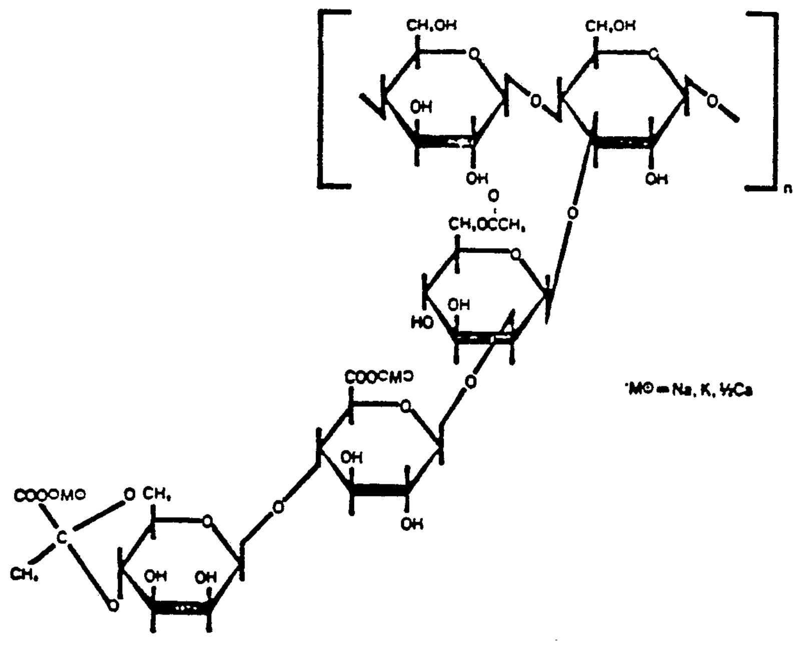

$^{*}M^{\oplus}$ = Na, K, ½Ca

das bei der Fermentation von Xanthomonas campestris entsteht. Die erfindungsgemäßen Gele werden hergestellt, indem man einer 1 %-igen Lösung von Benzylalkohol in entmineralisiertem Wasser, die außerdem die üblichen Formulierungshilfsstoffe enthält, bei 60°C unter schnellem Rühren 2,5 - 17,5 % Celluloseether als Gelbildner und 0,1 - 1,5 % Xanthan Gum zusetzt, auf 30°C abkühlt, 0,05 - 1 %, vorzugsweise 0,1 - 1 % Azolderivat zusetzt und die Mischung auf Zimmertemperatur abkühlt.

**Beispiel 1**

In 94,89 kg entmineralisiertem Wasser werden 1 kg Benzylalkohol, 0,1 kg Saccharin, 0,01 kg Na-Citrat primär gelöst und auf 60°C erwärmt. Bei 60°C werden unter schnellem Rühren 2,5 kg Hydroxypropylcellulose (M.G. 1 000 000) und 0,5 kg Xanthan Gum eingearbeitet und auf 30°C abgekühlt. Bei 30°C wird 1,0 kg Clotrimazol ebenfalls unter schnellem Rühren zugegeben. Die Mischung wird auf Raumtemperatur abgekühlt.

In analoger Weise werden Rezepturen der nachstehenden Beispiele verarbeitet.

**Beispiel 2**

| | |
|---|---|
| Bifonazol Wirkstoff micron. | 1,00 |
| Benzylalkohol | 1,00 |
| Hydroxypropylcellulose (M.G. ca. 1 000 000) | 2,5 |
| Xanthan Gum (M.G. ca 2 000 000) | 0,5 |
| Süßstoff | 0,1 |
| Aroma | 0,2 |
| Wasser entmineralisiert ad | 100 |

**Beispiel 3**

| | |
|---|---|
| Lombazol Wirkstoff micron. | 1,00 |
| Benzylalkohol | 1,00 |
| Hydroxypropylcellulose (M.G. 1 000 000) | 2,5 |
| Xanthan Gum (M.G. ca. 2 000 000) | 0,5 |
| Süßstoff | 0,1 |
| Aroma | 0,25 |
| Wasser entmineralisiert ad | 100 |

**Beispiel 4**

| | |
|---|---|
| Clotrimazol Wirkstoff micron. | 1,00 |
| Benzylalkohol | 1,00 |
| Hydroxypropylcellulose (M.G. 60 000) | 17,50 |
| Xanthan Gum (M.G. ca. 2 000 000) | 0,50 |
| Süßstoff | 0,20 |
| Wasser entmineralisiert ad | 100 |

**Patentansprüche**

1. Antimykotische Mittel in Gelform zur Behandlung von Pilzinfektionen der Mundhöhle, enthaltend Azolderivate und übliche Formulierungshilfsstoffe, dadurch gekennzeichnet, daß sie 2,5 % - 17,5 % Celluloseether als Gelbildner und 0,1 - 1,5 % Xanthan Gum als Stabilisator und/oder Gelbildner enthalten.

2. Antimykotische Gele nach Anspruch 1, dadurch gekennzeichnet, daß sie als Wirkstoff Clotrimazol der Formel

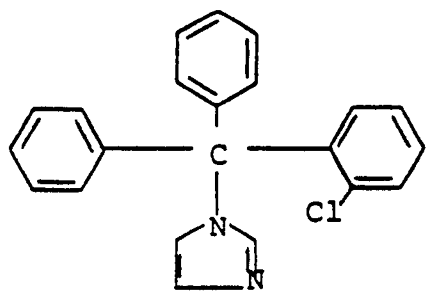

enthalten.

3. Antimykotische Gele nach Anspruch 1, dadurch gekennzeichnet, daß sie als Wirkstoff Bifonazol der Formel

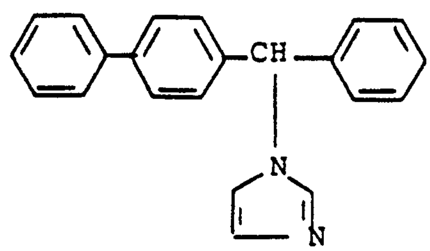

enthalten.

4. Antimykotische Gele nach Anspruch 1, dadurch gekennzeichnet, daß sie als Wirkstoff Lombazol der Formel

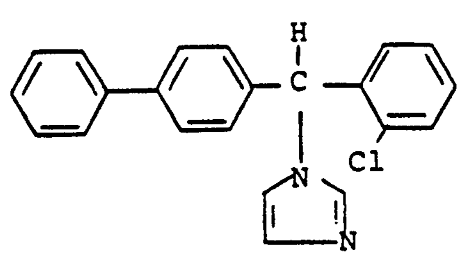

enthalten.

5. Antimykotische Gele nach Anspruch 1, dadurch gekennzeichnet, daß sie die antimykotischen Azolderivate in Mengen von 0,05 - 1 %, vorzugsweise von 0,1 - 1 %, enthalten.

6. Antimykotisches Gel nach Anspruch 1, dadurch gekennzeichnet, daß es als Gelbildner Hydroxypropyl cellulose enthält.

7. Antimykotisches Gel nach Anspruch 1, dadurch gekennzeichnet, daß die als Gelbildner verwendete Hydroxypropylcellulose ein Molgewicht zwischen 60 000 und 1 000 000 hat.

8. Antimykotisches Gel nach Anspruch 1, dadurch gekennzeichnet, daß es als Stabilisator und Gelbildner Xanthan Gum enthält.

9. Verfahren zur Herstellung von antimykotischen Mitteln in Gelform zur Behandlung von Pilzinfektionen der Mundhöhle, dadurch gekennzeichnet, daß man einer 1 %-igen Lösung von Benzylalkohol in entmineralisiertem Wasser, die außerdem die üblichen Formulierungshilfsstoffe enthält, bei 60°C unter schnellem Rühren 2,5 - 17,5 % Celluloseether als Gelbildner und 0,1 - 1,5 % Xanthan Gum zusetzt, auf 30°C abkühlt, 0,05 - 1 %, vorzugsweise 0,1 - 1 % Azolderivat zusetzt und die Mischung auf Zimmertemperatur abkühlt.

10. Verfahren nach Anspruch 9, dadurch gekennzeichnet, daß man als Celluloseether Hydroxypropylcellulose zusetzt.

**Claims**

1. Antimycotic agents in gel form for the treatment of fungal infections of the oral cavity, containing azole derivatives and customary formulating auxiliaries, characterized in that they contain 2.5 % - 17.5 % of cellulose ethers as the gel-forming agent and 0.1 - 1.5 % of xanthan gum as the stabiliser and/or gel-forming agent.

2. Antimycotic gels according to Claim 1, characterized in that they contain, as the active compound, clotrimazole of the formula

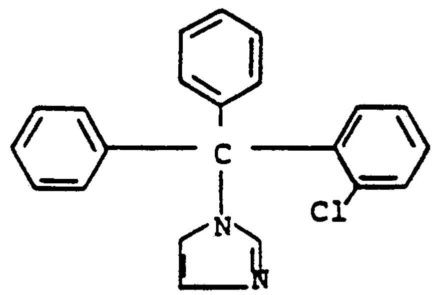

3. Antimycotic gels according to Claim 1, characterised in that they contain as the active compound, bifonazole of the formula

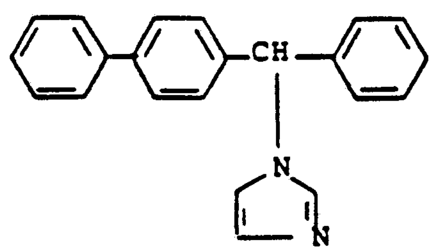

4. Antimycotic gels according to Claim 1, characterized in that they contain, as the active compound, lombazole of the formula

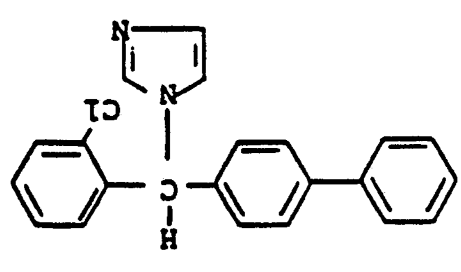

5. Antimycotic gels according to Claim 1, characterized in that they contain antimycotic azole derivatives in amounts 0.05 - 1 %, preferably 0.1 - 1 %.

6. Antimycotic gel according to Claim 1, characterized in that it contains hydroxypropylcellulose as the gelforming agent.

7. Antimycotic gel according to Claim 1, characterized in that the hydroxypropylcellulose used as the gel-forming agent has a molecular weight between 60,000 and 1,000,000.

8. Antimycotic gel according to Claim 1, characterized in that it contains xanthan gum as the stabiliser and gelforming agent.

9. Process for the preparation of antimycotic agents in gel form for the treatment of fungal inspections of the

oral cavity, characterized in that 2.5 - 17.5 % of cellulose ethers as the gel-forming agent and 0.1 - 1.5 % of xanthan gum are added to a 1 % strength solution of benzyl alcohol in demineralised water which also contains the customary formulating auxiliaries, at 60° C and with vigorous stirring, the mixture is cooled to 30° C, 0.05 - 1 %, preferably 0.1 - 1 %, of azole derivative is added and the mixture is cooled to room temperature.

10. Process according to claim 9, characterized in that hydroxypropylcellulose is added as the cellulose ether.

**Revendications**

1. Agents antimycotiques en forme de gel pour le traitement des infections fongiques de la cavité buccale, contenant des dérivés azoliques et des auxiliaires courants de formulation, caractérisés en ce qu'ils contiennent 2,5 % à 17,5 % d'éther de cellulose comme formateur de gel et 0,1 à 1,5 % de gomme xanthane comme stabilisateur et/ou comme formateur de gel.

2. Gels antimycotiques selon la revendication 1, caractérisés en ce qu'ils contiennent comme matière active du Clotrimazol:

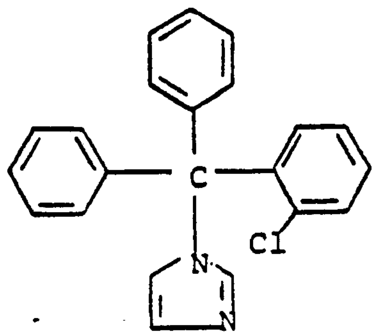

3. Gels antimycotiques selon la revendication 1, caractérisés en ce qu'ils contiennent comme matière active du Bifonazol de formule

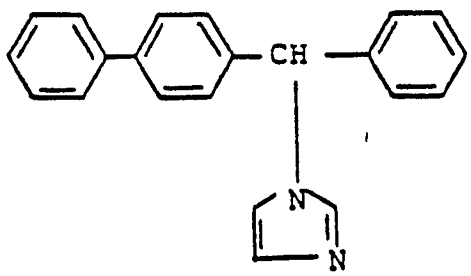

4. Gels antimycotiques selon la revendication 1, caractérisés en ce qu'ils contiennent comme matière active du Lombazol de formule:

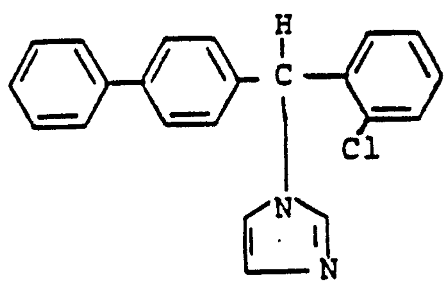

5. Gels antimycotiques selon la revendication 1, caractérisés en ce qu'ils contiennent les dérivés azoliques antimycotiques en des quantités de 0,05 à 1 %, de préférence de 0,1 à 1 %.

6. Gel antimycotique selon la revendication 1, caractérisé en ce qu'il contient comme formateur de gel de l'hydroxypropylcellulose.

7. Gel antimycotique selon la revendication 1, caractérisé en ce que l'hydroxypropylcellulose utilisée comme formateur de gel a un poids moléculaire entre 60 000 et 1 000 000.

8. Gel antimycotique selon la revendication 1, caractérisé en ce qu'il contient de la gomme xanthane comme stabilisateur et formateur de gel.

9. Procédé de fabrication d'agents antimycotiques en forme de gel pour le traitement d'infections fongiques de la cavité buccale, caractérisé en ce qu'on ajoute à une solution à 1 % d'alcool benzylique dans de l'eau déminéralisée, qui contient en outre les auxiliaires usuels de formulation, à 60° C et sous agitation rapide, 2,5 à 17,5 % d'éther de cellulose comme formateur de gel et 0,1 à 1,5 % de gomme xanthane, on refroidit à 30° C, on ajoute 0,05 à 1 %, de préférence 0,1 à 1 % de dérivé azolique et l'on refroidit le mélange à la température ordinaire.

10. Procédé selon la revendication 9, caractérisé en ce qu'en tant qu'éther de cellulose on ajoute de l'hydroxypropylcellulose.